# EUROPÄISCHE PATENTSCHRIFT

(11) **EP 0 673 377 B1**
(45) Veröffentlichungstag und Bekanntmachung des Hinweises auf die Patenterteilung: **26.02.1997**
(21) Anmeldenummer: 94901897.2
(22) Anmeldetag: 27.11.1993
(51) Int. Cl.: C07D 487/04, A61K 31/505

(54) **NEUE TRIAZOLOCHINAZOLINE, IHRE HERSTELLUNG UND VERWENDUNG**
NEW TRIAZOLOQUINAZOLINES, THEIR PRODUCTION AND THEIR USE
NOUVELLES TRIAZOLOQUINAZOLINES, LEUR PRODUCTION ET LEUR UTILISATION

(30) Priorität: 10.12.1992 DE 4241563
(43) Veröffentlichungstag der Anmeldung: 27.09.1995
(73) Patentinhaber: BASF AKTIENGESELLSCHAFT, 67056 Ludwigshafen (DE)
(72) Erfinder: SCHLECKER, Rainer, D-67281 Bissersheim (DE); TREIBER, Hans-Joerg, D-68782 Bruehl (DE); BEHL, Berthold, D-67063 Ludwigshafen (DE); HOFMANN, Hans, Peter, D-67117 Limburgerhof (DE)
(86) Internationale Anmeldenummer: EP9303331
(87) Internationale Veröffentlichungsnummer: WO9413672

(56) Entgegenhaltungen:
- US-A- 4 128 644
- JOURNAL OF MEDICINAL CHEMISTRY Bd. 34 , 1991 , WASHINGTON US Seiten 281 - 290 J.E. FRANCIS ET AL. 'Synthesis and benzodiazepine binding activity of a series of novel [1,2,4]triazolo[1,5-c]quin azolin-5(6h)-0nes'

## Beschreibung

Die vorliegende Erfindung betrifft neue Triazolochinazoline, Verfahren zu deren Herstellung sowie deren Verwendung zur Bekämpfung von Krankheiten.

Es sind bereits Pyrazolo- und Triazolochinazoline bekannt, die antiallergische und entzündungshemmende Eigenschaften besitzen (EP 80.176, US 4.053.600, US 4.128.644). Weiter sind Pyrazolochinazoline bekannt, die sich darüberhinaus zur Behandlung von Thrombose und neurologischen Störungen eignen (US 5.153.196). Weiterhin ist ein 1,2,4-Triazolo[1,5-c]chinazolin-5-on beschrieben, welches in 2-Position mit -COOE+ substituiert ist. Es besitzt FNZ (Flunitrazeparn)-Bindungsaffinität (J.Med.Chem., 1991, Vol.34, Nr.1, Seite 284).

Es wurde nun gefunden, daß Triazolochinazoline der Formel I 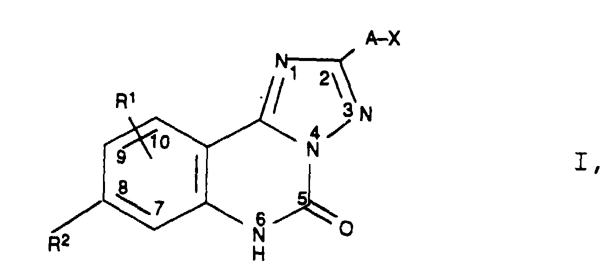 worin
- A: eine C₁₋₅-Alkylengruppe
- X: eine Carboxylgruppe, die in Form ihres Salzes mit einem physiologisch verträglichen Amin- oder Metallkation vorliegen kann; den Rest 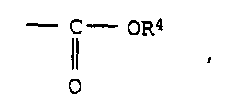 wobei R⁴ einen C₁₋₈-Alkylrest, eine Cycloalkylgruppe mit 3 bis 8 Kohlenstoffatomen im Ring, einen Benzylrest, einen der Reste -(CH₂)ₙ-O-R⁵ oder 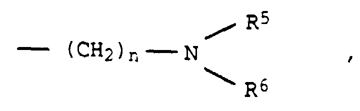 in denen n für die Zahl 2, 3 oder 4 und
R⁵ und R⁶ für eine C₁₋₃-Alkylgruppe stehen; eine Hydroxyl-, C₁₋₄-Hydroxyalkyl-, C₁₋₄-Alkylcarbonyl-, Nitrilo-C₁₋₄-alkyl-, Tetrazolyl- oder eine Carbonylaminotetrazolgruppe darstellt, und
R¹ und R², die gleich oder verschieden sein können, Wasserstoff-, Fluor-, Chlor- oder Bromatome, Trifluormethyl-, Cyano-, Nitro-, Amino-, C₁₋₅-Alkyl-, Mono- oder Di-C₁₋₅-alkylaminogruppen, einen C₁₋₆-Alkylthio-, C₁₋₆-Alkylsulfenyl-, C₁₋₆-Alkylsulfonyl-, Aminosulfonyl-, Di-C₁₋₆-alkylaminosulfonylrest bedeuten oder
R¹ und R² zusammen eine Methylen- oder Ethylendioxygruppe oder eine geradkettige C₃₋₅-Alkylengruppe bedeuten, ein anderes Wirkungsspektrum zeigen.

Bevorzugt sind die Verbindungen der Formel I, in denen A und X die angegebene Bedeutung besitzen und R¹ ein Wasserstoff- oder Chloratom oder eine Trifluormethyl-, Nitro- oder C₁₋₃-Alkylgruppe und R² ein Chloratom oder eine Trifluormethyl-, Nitro- oder C₁₋₃-Alkylgruppe darstellen oder R¹ und R² zusammen eine geradkettige C₃₋₅-Alkylengruppe bedeuten.

Als Reste A-X in Position 2 der oben aufgeführten 1,2,4-Triazolo-[1,5-c]chinazolin-5-one seien folgende beispielhaft genannt:

Acetyl, 2-Propionyl, 3-Propionyl, 4-Buturyl, 3-Buturyl, 2-Buturyl, 5-Valeryl, 4-Valeryl, 3-Valeryl sowie ihre jeweiligen Methyl-, Ethyl-, Propyl-, Isopropyl-, Butyl-, Pentyl-, Hexyl-, Heptyl-, Cyclopropyl-, Cyclobutyl-, Cyclopentyl-, Cyclohexyl-, Cycloheptyl- und Cyclooctylester oder ihre Amide, wie Methylamide, Dimethylamide, Ethylamide, Diethylamide, Propylamide, Butylamide und Benzylamide;

Hydroxymethyl, 1-Hydroxyethyl, 2-Hydroxyethyl, 1-Hydroxypropyl, 2-Hydroxypropyl, 3-Hydroxypropyl, 1-Hydroxybutyl, 2-Hydroxybutyl, 3-Hydroxybutyl, 4-Hydroxybutyl, Hydroxypentyl, Hydroxyheptyl;

Methoxymethyl, 1-Methoxypropyl, 2-Methoxypropyl, 3-Methoxypropyl, Methoxybutyl, Ethoxymethyl, 1-Ethoxypropyl, 2-Ethoxypropyl, 3-Ethoxypropyl, Ethoxybutyl, Oxomethyl, 1-Oxoethyl, 2-Oxoethyl, 1-Oxopropyl, 2-Oxopropyl, 3-Oxopropyl, 1-Oxobutyl, 2-Oxobutyl, 3-Oxobutyl, 4-Oxobutyl, 1-Oxopentyl, 2-Oxopentyl, 3-Oxopentyl, 4-Oxopentyl,

Cyanomethyl, 1-Cynanoethyl, 1-Cyanopropyl, 2-Cyanopropyl, 3-Cyanopropyl, 1-Cyanobutyl, 2-Cyanobutyl, 3-Cyanobutyl, 4-Cyanobutyl Als Grundkörper ohne den Substituenten AX seien folgende genannt:
10-Chlor-1,2,4-triazolo[1,5-c]chinazolin-5-on
8-Chlor-1,2,4-triazolo[1,5-c]chinazolin-5-on
7-Chlor-1,2,4-triazolo[1,5-c]chinazolin-5-on
8,10-Dichlor-1,2,4-triazolo[1,5-c]chinazolin-5-on
10-Brom-1,2,4-triazolo[1,5-c]chinazolin-5-on
9-Brom-1,2,4-triazolo[1,5-c]chinazolin-5-on
8-Brom-1,2,4-triazolo[1,5-c]chinazolin-5-on
7-Brom-1,2,4-triazolo[1,5-c]chinazolin-5-on
8,10-Dibrom-1,2,4-triazolo[1,5-c]chinazolin-5-on
10-Jod-1,2,4-triazolo[1,5-c]chinazolin-5-on
9-Jod-1,2,4-triazolo[1,5-c]chinazolin-5-on
8-Jod-1,2,4-triazolo[1,5-c]chinazolin-5-on
7-Jod-1,2,4-triazolo[1,5-c]chinazolin-5-on
8,10-Dijod-1,2,4-triazolo[1,5-c]chinazolin-5-on
10-Jod-8-chlor-1,2,4-triazolo[1,5-c]chinazolin-5-on
9-Trifluormethyl-1,2,4-triazolo[1,5-c]chinazolin-5-on
9-Trifluormethyl-8-nitro-1,2,4-triazolo[1,5-c]chinazolin-5-on
9-Trifluormethyl-8-methansulfonyl-1,2,4-triazolo[1,5-c]chinazolin-5-on
9-Methyl-8-nitro-1,2,4-triazolo[1,5-c]chinazolin-5-on
9-Ethyl-8-nitro-1,2,4-triazolo[1,5-c]chinazolin-5-on
9-Cyano-8-nitro-1,2,4-triazolo[1,5-c]chinazolin-5-on
10-Cyano-1,2,4-triazolo[1,5-c]chinazolin-5-on
9-Cyano-1,2,4-triazolo[1,5-c]chinazolin-5-on
8-Cyano-1,2,4-triazolo[1,5-c]chinazolin-5-on
7-Cyano-1,2,4-triazolo[1,5-c]chinazolin-5-on
9-Cyano-8-methansulfonyl-1,2,4-triazolo[1,5-c]chinazolin-5-on
9-Cyano-8-trifluormethyl-1,2,4-triazolo[1,5-c]chinazolin-5-on
10-Methyl-1,2,4-triazolo[1,5-c]chinazolin-5-on
8-Methyl-1,2,4-triazolo[1,5-c]chinazolin-5-on
7-Methyl-1,2,4-triazolo[1,5-c]chinazolin-5-on
9-Methyl-1,2,4-triazolo[1,5-c]chinazolin-5-on
9,10-Dimethyl-1,2,4-triazolo[1,5-c]chinazolin-5-on
7,8-Dimethyl-1,2,4-triazolo[1,5-c]chinazolin-5-on
9,10-Tetramethylen-1,2,4-triazolo[1,5-c]chinazolin-5-on
7,8-Tetramethylen-1,2,4-triazolo[1,5-c]chinazolin-5-on
9,10-Trimethylen-1,2,4-triazolo[1,5-c]chinazolin-5-on
8,9-Trimethylen-1,2,4-triazolo[l,5-c]chinazolin-5-on
7,8-Trimethylen-1,2,4-triazolo[1,5-c]chinazolin-5-on
9,10-Pentamethylen-1,2,4-triazolo[1,5-c]chinazolin-5-on
8,9-Pentamethylen-1,2,4-triazolo[1,5-c]chinazolin-5-on
7,8-Pentamethylen-1,2,4-triazolo[1,5-c]chinazolin-5-on
10-Isopropyl-1,2,4-triazolo[1,5-c]chinazolin-5-on
9-Isopropyl-1,2,4-triazolo[1,5-c]chinazolin-5-on
8-Isopropyl-1,2,4-triazolo[1,5-c]chinazolin-5-on
7-Isopropyl-1,2,4-triazolo[1,5-c]chinazolin-5-on
9,10 Benzo-1,2,4-triazolo[1,5-c]chinazolin-5-on
8-Sulfonylamido-1,2,4-triazolo[1,5-c]chinazolin-5-on
8-Sulfonylamido-9-trifluormethyl-1,2,4-triazolo[1,5-c]chinazolin-5-on
10-Methylthio-1,2,4-triazolo[1,5-c]chinazolin-5-on
9-Methylthio-1,2,4-triazolo[1,5-c]chinazolin-5-on
8-Methylthio-1,2,4-triazolo[1,5-c]chinazolin-5-on
7-Methylthio-1,2,4-triazolo[1,5-c]chinazolin-5-on
9-Methylthio-8-nitro-1,2,4-triazolo[1,5-c]chinazolin-5-on
8-Trifluormethansulfonyl-1,2,4-triazolo[1,5-c]chinazolin-5-on
9-Trifluormethansulfonyl-8-nitro-1,2,4-triazolo[1,5-c]chinazolin-5-on
10-Dimethylamino-1,2,4-triazolo[1,5-c]chinazolin-5-on
9-Dimethylamino-1,2,4-triazolo[1,5-c]chinazolin-5-on
8-Dimethylamino-1,2,4-triazolo[1,5-c]chinazolin-5-on
8,9-Methylendioxy-1,2,4-triazolo[1,5-c]chinazolin-5-on
9,10-Methylendioxy-1,2,4-triazolo[1,5-c]chinazolin-5-on
9-Butoxy-1,2,4-triazolo[1,5-c]chinazolin-5-on
8-Butoxy-9-cyano-1,2,4-triazolo[1,5-c]chinazolin-5-on

Die Herstellung der Verbindungen der Formel I erfolgt durch eine intramolekulare Kondensationsreaktion eines Hydrazinochinazolins der Formel II, 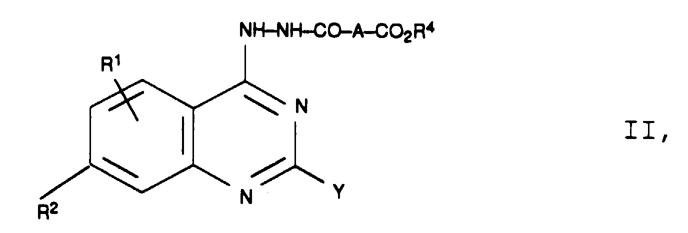 in der A, R¹ und R² die für Formel I angegebenen Bedeutungen haben, R⁴ ein C₁₋₈-Alkylrest, eine Cycloalkylgruppe mit 3 bis 8 Kohlenstoffatomen im Ring, ein Benzylring oder der Rest -(CH₂)-OR⁵ ist und Y eine Hydroxylgruppe oder ein Brom- oder Chloratom darstellt, vorzugsweise in Anwesenheit eines Dehydratisierungsmittels, insbesondere von Phosphoroxychlorid, Polyphosphorsäure oder Essigsäure, gegebenenfalls in einem inerten Lösungsmittel, wie Toluol, Chlorbenzol, Xylol oder überschüssiger Essigsäure, bei Temperaturen von 50 bis 150°C, und zwar vorzugsweise bei der Rückflußtemperatur der Reaktionsmischung.

Die so erhaltenen Ester können anschließend verseift und die freien Säuren mit einem Amin oder einem Metallkation in physiologisch verträgliche Salze überführt werden. Die freien Säuren können auch in die Hydroxyalkylverbindungen (A-X = Hydroxyalkyl) reduziert oder nach bekannter Methode in die Nitrile, Tetrazolamino- und Carbamoylverbindungen überführt werden.

Die Ester der Formel I können auch einem üblichen Umesterungsverfahren entsprechend den Bedeutungen für den Rest R⁴ unterworfen werden.

Die Herstellung von Verbindungen der Formel I, in denen X eine Carboxylgruppe darstellt, erfolgt durch Hydrolyse der entsprechenden Ester vorzugsweise unter alkalischen Bedingungen, beispielsweise in Gegenwart eines Alkalimetallhydroxids oder von Natriumhydrogencarbonat, in einem Lösungsmittel, wie Wasser, einem niederen Alkohol, Tetrahydrofuran oder Mischungen derselben. Die so erhaltenen organischen Säuren werden gegebenenfalls in ein physiologisch verträgliches Amin- oder Metallsalz überführt. Darunter versteht man insbesondere Salze der Alkalimetalle, wie Natrium und Kalium, der Erdalkalimetalle, wie Calcium, sonstiger Metalle, wie Aluminium, sowie Salze von organischen Basen, wie Morpholin, Piperidin, Mono-, Di- und Triethanolamin oder Tris(hydroxymethyl)aminomethan, die dem Fachmann allgemein bekannt sind.

Carbonsäuren der Formel I können weiterhin hergestellt werden durch Hydrogenolyse der entsprechenden Benzylester nach bekannten Methoden, wie sie beispielsweise in Houben-Weyl Bd. IV/1c S. 381 ff beschrieben sind. Die Reaktion erfolgt in Anwesenheit eines Katalysators, wie Platin, Palladium oder Nickel, zweckmäßig auf einem Träger, insbesondere Kohle, in einem Lösungsmittel, wie einem niederen Alkohol, insbesondere Methanol, Essigsäure oder einem Dialkylformamid, insbesondere Dimethylformamid, zwischen Temperaturen von 0°C bis zum Siedepunkt des Lösungsmittels, und vorzugsweise unter nur leicht erhöhtem Druck.

Amide der Formel I, in der X für eine Carbamoylgruppe steht, werden durch Umsetzung der Ester mit Ammoniak oder Aminen in Gegenwart eines Lösungsmittels wie Wasser, eines niederen Alkohols, einer wäßrig-alkoholischen Lösung oder Dialkylformamid bei Temperaturen zwischen 0°C und der Rückflußtemperatur des Systems erhalten.

Durch Behandlung primärer Amide mit einem Dehydratisierungsmittel, wie Phosphorpentoxid, Phosphoroxychlorid oder Thionylchlorid, erhält man die Nitrile der Verbindungen der Formel I, worin X eine Nitrilgruppe bedeutet. Die Reaktion wird im allgemeinen mit einem Überschuß des Dehydratisierungsmittels bei der Rückflußtemperatur der Mischung durchgeführt. Gegebenenfalls kann die Reaktion in Gegenwart eines inerten Lösungsmittels, wie Benzol oder Ethylenchlorid, durchgeführt werden.

Die Synthese von Verbindungen der Formel I, in denen X für einen Tetrazolrest steht, erfolgt nach an sich bekannten Methoden, wie sie beispielsweise in Synth. 1973, 80 beschrieben sind, durch Umsetzung der Amide mit Stickstoffwasserstoffsäure oder einem ihrer Salze, beispielsweise mit Alkali- oder Erdalkaliaziden, gegebenenfalls in Anwesenheit von Lewissäuren wie Aluminium- und Zinnchlorid oder von Ammoniumchlorid. Bevorzugt ist die Kombination von Natriumazid mit Ammoniumchlorid. Im allgemeinen wird die Reaktion in Anwesenheit eines inerten Lösungsmittels wie Benzol, Tetrahydrofuran oder Dimethylformamid bei Temperaturen zwischen Raumtemperatur und 150°C durchgeführt. Die Tetrazolylverbindungen sind stark sauer und können in üblicher Weise in ein Salz mit einem physiologisch verträglichen Amin- oder Metallkation überführt werden.

Die Reduktion von Carbonsäuren, insbesondere eines Esters einer Verbindung der Formel I, nach bekannten Verfahren, beispielsweise mit Hilfe eines komplexen Metallhydrids, wie Lithiumborhydrid, in Gegenwart eines Ethers, wie Tetrahydrofuran, als Lösungsmittel liefert die Hydroxymethylverbindungen der Formel I (X = CH₂OH). Die Reduktion wird vorzugsweise beim Siedepunkt der Reaktionsmischung durchgeführt.

Verbindungen der Formel I mit einem Carbonylaminotetrazolrest für X (X = CO-NH-CHN₄) können nach bekannten Methoden durch Kondensation der zugrundeliegenden Carbonsäure mit 5-Aminotetrazol der Formel III. 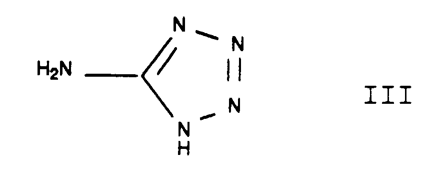 erhalten werden. Die Reaktion wird in der Regel in einem inerten Lösungsmittel, wie beispielsweise Methylenchlorid, Dioxan, Tetrahydrofuran oder Dimethylformamid, bevorzugt in Anwesenheit eines aus der Peptidchemie bekannten Kondensationsreagenzes, wie N,N'-Carbonyldiimidazol oder N,N'-Dicyclohexylcarbodiimid, bei Temperaturen von 20°C bis 120°C durchgeführt.

In analoger Weise können auch Verbindungen der Formel I, in der X für einen gegebenenfalls substituierten Carbamoylrest steht, aus den entsprechenden Säuren hergestellt werden.

Falls in den Ausgangsverbindungen die Substituenten R¹ und R² noch nicht vorhanden sind, können diese auch nachträglich eingeführt werden. Das kann durch eine elektrophile aromatische Substitution einer erhaltenen Verbindung der Formel I, in der R¹ und/oder R² Wasserstoffatome bedeuten, nach an sich bekannten Methoden geschehen, wie sie beispielsweise in Houben-Weyl Bd. X/1, S. 471 ff, Bd. IX, S. 572 ff, und Bd. V/3, S. 873, beschrieben sind. So kann die Nitrierung mit einer Mischung aus Schwefel- und Salpetersäure bei Raumtemperatur durchgeführt werden, die Sulfonierung beispielsweise mit Chlorsulfonsäure zwischen Raumtemperatur und 150°C, die Chlorierung mit Sulfurylchlorid bei 20°C bis 100°C.

Die Herstellung der Ausgangsverbindungen der Formel II erfolgt in an sich bekannter Weise durch Kondensation von einem Hydrazinochinazolin der Formel IV 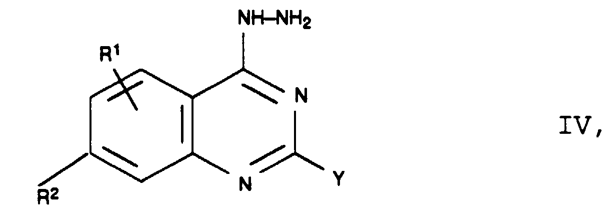 in der R¹, R² und Y die oben genannten Bedeutungen haben, mit einem Dicarbonsäureesterhalogenid oder einem Dicarbonsäurediester. Bei Verwendung eines Esterhalogenids, bevorzugt eines Chlorids, erfolgt die Reaktion zweckmäßig bei Temperaturen von -30°C bis 70°C, vorzugsweise bei Raumtemperatur, in einem inerten Lösungsmittel wie Dimethylformamid, Dioxan, Tetrahydrofuran oder Methylenchlorid. Die Umsetzung wird bevorzugt in Anwesenheit von tertiären organischen Basen, wie Triethylamin oder Pyridin, durchgeführt.

Die Umsetzung von IV mit Estern kann mit oder ohne Lösungsmittel, wie Toluol, Chlorbenzol oder Diphenylether, bei einer Temperatur von etwa 20°C bis zur Rückflußtemperatur der Mischung durchgeführt werden.

Ein weiteres Verfahren zur Herstellung von Ausgangsverbindungen der Formel II besteht in der Reaktion von einem Acylhydrazin der Formel V 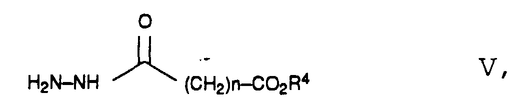 in der R⁴ die oben genannte Bedeutung hat, mit einem Chinazolin der Formel VI 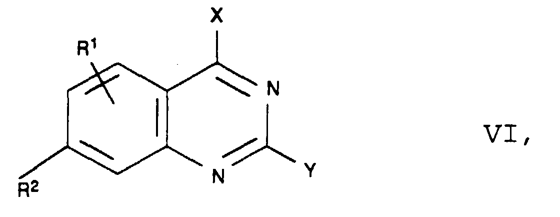 in der R¹, R² und Y die oben genannte Bedeutung haben und X eine nucleofuge Abgangsgruppe, bevorzugt ein Halogenatom, wie beispielsweise Chlor, ist. Die Reaktion wird zwischen 0°C und 50°C in einem inerten Lösungsmittel wie Ethanol, Methylenchlorid, Toluol, Tetrahydrofuran oder Dimethylformamid, vorzugsweise mit einem Überschuß an V, durchgeführt.

Die Synthese der Verbindungen der Formeln IV und VI ist in EP 80 176 beschrieben.

Die erfindungsgemäßen Verbindungen I eignen sich als Arzneimittelwirkstoff für die Human- und Veterinärmedizin und können zur Herstellung von Arzneimitteln zur Behandlung neurodegenerativer Erkrankungen und neutoxischer Störungen des zentralen Nervensystems sowie zur Herstellung von Spasmolytika, Antiepileptika, Anxiolytika und Antidepressiva verwendet werden.

Die pharmakologische Wirksamkeit der erfindungsgemäßen Verbindungen I wurde an isoliertem Membranmaterial von Rattengroßhirnen untersucht. Hierzu wurde das Membranmaterial in Gegenwart der erfindungsgemäßen Verbindungen mit den radioaktiv markierten Substanzen ³H-2-Amino-3-hydroxy-5-methyl-4-isoxazol-propionsäure (³H-AMPA) und ³H-2-Amino-3-hydroxy-5-methyl-4-isoxazol-propionsäure (³H-AMPA) und ³H-5,7-Dichlorkynurensäure behandelt, wobei diese an spezifische Rezeptoren (AMPA- bzw. NMDA-Rezeptor (N-Methyl-D-aspartat)) binden. Anschließend wurde durch Scintillationszählung die Radioaktivität der behandelten Membrane gemessen. Über die gebundene Radioaktivität ließen sich die Mengen an gebundener ³H-AMPA und ³H-5,7-Dichlorkynurensäure bzw. jeweils die verdrängten Mengen dieser radioaktiv markierten Substanzen bestimmen. Die sich heraus ergebende Dissoziationskonstante K_{I} (I = Inhibitor), welche ein Maß für die Verdrängungswirkung des erfindungsgemäßen Wirkstoffs ist, wurde durch iterative nichtlineare Regressionsanalyse mit dem Statistical Analysis System (SAS) an einem IBM-Rechner, ähnlich dem Programm "Ligand" von P.J. Munson oder D. Rodbard (Analytical Biochem. 107, 220 (1980), Ligand: Versatile Computerized Approach for Charakterization of Ligand Binding Systems) ermittelt.

Folgende In-Vitro-Untersuchungen wurden durchgeführt:

### 1. Bindung von ³H-2-Amino-3-hydroxy-5-methyl-4-isoxanolpropionsäure (³H-AMPA)

Für die Präparation der Membranen wurden frisch entnommene Rattengroßhirne zusammen mit dem ca. 15fachen Volumen einer Pufferlösung A aus 30 mM α α,α-Tris-(hydroxymethyl)-methylamin-Hydrochlorid (TRIS-HCl) und 0,5 mM Ethylendiamintetraessigsäure (EDTA) - pH 7,4 - mittels eines Ultra-TURRAX-Rührers homogenisiert. Die Suspension wurde 20 min bei 48 000 g zentrifugiert. Nach Abtrennung der überstehenden Flüssigkeit wurde das im Bodensatz enthaltene proteinhaltige Membranmaterial dreimal durch Suspendieren in der Pufferlösung A und anschließendes, jeweils 20minütiges Zentrifugieren bei 48 000 g gewaschen. Danach wurde das Membranmaterial in einem 15fachen Volumen der Pufferlösung A suspendiert und 30 Minuten bei 37°C inkubiert. Anschließend wurde das Proteinmaterial zweimal durch Zentrifugieren und Suspendieren gewaschen und bis zur Verwendung bei -70°C eingefroren.

Für den Bindungstest wurde das bei 37°C aufgetaute Proteinmaterial zweimal durch Zentrifugieren bei 48 000 g (20 Minuten) und anschließendes Suspendieren in einer Pufferlösung B aus 50 mM TRIS-HCl, 0,1 M Kaliumthiocyanat und 2,5 mM Calciumchlorid - pH 7,1 - gewaschen. Anschließend wurden 0,25 mg Membranmaterial, 0,1 µCi ³H-AMPA (60 Ci/mmol) sowie Verbindung I in 1 ml Pufferlösung B gelöst und 60 Minuten auf Eis inkubiert. Die inkubierte Lösung wurde über einen CF/B-Filter (Firma Whathman), der zuvor mindestens 2 Stunden mit einer 0,5%igen wäßrigen Lösung von Polyethylenimin behandelt worden war, filtriert. Anschließend wurde das Filtrat mit 5 ml kalter Pufferlösung B gewaschen, um gebundene und freie ³H-AMPA voneinander zu trennen. Nach Messung der Radioaktivität der gebundenen ³H-AMPA im Membranmaterial durch Scintillationszählung wurde durch Auswertung der Verdrängungskurven mittels Regressionsanalyse der K_{I}-Wert bestimmt.

Es wurden folgende Ergebnisse erhalten:

| Beispiel Nr. | AMPA-Bindung Ki [µM] |
|---|---|
| 13 | 3,4 |
| 14 | 0,6 |
| 15 | 1,0 |
| 18 | 0,6 |
| 19 | 0,7 |
| 20 | 0,6 |

### 2. Bindung von ³H-5,7-Dichlorkynurensäure

Für die Präparation des Membranmaterials wurden frisch entnommene Rattengroßhirne zusammen mit dem ca. 10fachen Volumen einer Pufferlösung A' aus 50 mM TRIS-HCl und 10 mM EDTA - pH7,4 - homogenisiert. Die Suspension wurde 20 Minuten bei 48 000 g zentrifugiert. Nach Abtrennung der überstehenden Flüssigkeit wurde das im Bodensatz enthaltene Membranmaterial zweimal durch Suspendieren in der Pufferlösung A' und anschließendes jeweils 20 minütiges Zentrifugieren und Suspendieren gewaschen. Nach erneutem Suspendieren der Membrane in der Pufferlösung A' und Einfrieren in flüssigem Stickstoff wurde die Suspension wieder bei 37°C aufgetaut und nach einem weiteren Waschvorgang 15 Minuten bei 37°C inkubiert.

Anschließend wurde das Proteinmaterial viermal durch Zentrifugieren und Suspendieren gewaschen und bis zur Verwendung bei -70°C eingefroren.

Für den Bindungstest wurde das bei 37°C aufgetaute Proteinmaterial zweimal durch Zentrifugieren bei 48 000 g (20 Minuten) und anschließendes Suspendieren in einer Pufferlösung B' aus 50 mM TRIS-HCl - pH 7,4 - gewaschen. Anschließend wurden 0,15 mg Membranmaterial, 0,3 µCi ³H-5,7-Dichlorkynurensäure (16 Ci/mmol) sowie Verbindung I in 1 ml Pufferlösung B' gelöst und 30 Minuten auf Eis inkubiert. Die inkubierte Lösung wurde 2 Minuten bei 150 000 g zentrifugiert. Nach Abtrennung der überstehenden Flüssigkeit wurden die Bodensätze zweimal mit je 1,5 ml kalter Pufferlösung B' suspendiert. Nach Messung der Radioaktivität der an die Membranen gebundenen ³H-5,7-Dichlorkynurensäure im Bodensatz ergab sich der K_{I}-Wert durch Auswertung der Verdrängungskurven mittels der Regressionsanalyse.

Es wurden folgende Ergebnisse erhalten:

| Beispiel Nr. | Bindung von Dichlorkynurensäure Ki [µM] |
|---|---|
| 4 | 1,75 |
| 13 | 0,5 |
| 14 | 0,17 |
| 15 | 0,4 |
| 16 | 0,1 |
| 18 | 0,65 |
| 19 | 0,3 |
| 20 | 0,4 |
| 21 | 0,8 |

Die Herstellung der Arzneimittelzubereitungen erfolgt in üblicher Weise, z.B. durch Vermischen des Wirkstoffes mit den anderen üblichen Trägerstoffen und Verdünnungsmitteln.

Die Arzneimittelzubereitungen können in verschiedenen Applikationsweisen verabreicht werden, wie peroral, parenteral, subkutan, intraperitonal und topisch. So sind Zubereitungsformen wie Tabletten, Emulsionen, Infusions- und Injektionslösungen, Pasten, Salben, Gele, Cremes, Lotionen, Puder und Sprays möglich.

Die erfindungsgemäßen Arzneimittelzubereitungen enthalten neben den üblichen Arzneimittelhilfsstoffen eine therapeutisch wirksame Menge der Verbindung I. Für die lokale äußere Anwendung, z.B. in Puder und Salben, können die Wirkstoffe in den üblichen Konzentrationen enthalten sein. In der Regel sind die Wirkstoffe in einer Menge von 0,001 bis 5 Gew.-%, vorzugsweise 0,02 bis 0,5 Gew.-% enthalten.

Bei der inneren Anwendung werden die Präparationen in Einzeldosen verabreicht. In einer Einzeldosis werden pro kg Körpergewicht 0,1 bis 50 mg, vorzugsweise 0,1 bis 10 mg Wirkstoff gegeben. Die Zubereitungen können täglich in einer oder mehreren Dosierungen je nach Art und Schwere der Erkrankungen verabreicht werden. Die Tagesdosis liegt in der Regel bei 0,1 bis 100 mg pro kg Körpergewicht bei oraler Gabe bzw. 0,01 bis 10 mg pro kg Körpergewicht bei parenteraler Gabe.

Entsprechend der gewünschten Applikationsart enthalten die erfindungsgemäßen Arzneimittelzubereitungen neben dem Wirkstoff die üblichen Trägerstoffe und Verdünnungsmittel. Für die lokale äußere Anwendung können pharmazeutisch-technische Hilfsstoffe, wie Ethanol, Isopropanol, oxethyliertes Ricinusöl, oxethyliertes hydriertes Ricinusöl , Polyacrylsäure, Polyethylenglykol, Polyethylenglykolstearat, ethoxylierte Fettalkohole, Paraffinöl, Vaseline und Wollfett verwendet werden. Für die innere Anwendung eignen sich z.B. Milchzucker, Propylenglykol, Ethanol, Stärke, Talk und Polyvinylpyrrolidon.

Ferner können Antioxidationsmittel wie Tocopherol und butyliertes Hydroxyanisol sowie butyliertes Hydroxytoluol, geschmacksverbessernde Zusatzstoffe, Stabilisierungs-, Emulgier- und Bleichmittel enthalten sein.

Die neben dem Wirkstoff in der Zubereitung enthaltenen Stoffe sowie die bei der Herstellung der pharmazeutischen Zubereitung verwendeten Stoffe müssen toxikologisch unbedenklich und mit dem jeweiligen Wirkstoff verträglich sein.

Die folgenden Beispiele erläutern die Erfindung näher.

### A. Herstellung von Ausgangsverbindungen

### Beispiel a

### 2-Chlor-4-N(N'-ethylsuccinylhydrazino)chinazolin

3 g 2-Chlor-4-hydrazinochinazolin wurden in 100 ml Methylenchlorid und 2 ml Triethylamin suspendiert und bei 0°C tropfenweise mit 3 g Bernsteinsäureethylesterchlorid versetzt. Die Mischung wurde über Nacht bei Raumtemperatur gerührt, der Niederschlag abgesaugt, mit Wasser gewaschen und getrocknet. Ausbeute: 4,1 g (82 %).

Analog wurden aus den entsprechenden Hydrazinochinazolinen und Säureesterchloriden hergestellt:
- 2-Chlor-4-N(N'-ethylglutarylhydrazino)chinazolin
- 2-Chlor-4-N(N'-ethylmalonylhydrazino)-9-nitrochinazolin
- 2-Chlor-8,9-dimethyl-4-N(N'-ethylsuccinylhydrazino)chinazolin

### B. Herstellung der Endprodukte

### Beispiel 1

### 3-(1,2,4-Triazolo[1,5-c]chinazolin-5-on-2)-propionsäureethylester

32 g 2-Chlor-4-N(N'-ethylsuccinylhydrazino)chinazolin wurden in 500 ml Essigsäure 2 h unter Rückfluß gekocht. Das Lösungsmittel wurde abdestilliert, der Rückstand mit Methanol behandelt, abgesaugt und getrocknet. Ausbeute: 19,4 g (68%); Fp. 208 - 210°C.

In analoger Weise wurden ausgehend von den entsprechenden Ausgangsverbindungen die nachstehenden Verbindungen hergestellt:
2. 4-(1,2,4-Triazolo[1,5-c]chinazolin-5-on-2)-buttersäuremethylester, Fp. 176 - 179°C
3. 2-(1,2,4-Triazolo[1,5-c]chinazolin-5-on-2)-essigsäureethylester, Fp. 206 - 210°C
4. 3-(8,9-Dimethyl-1,2,4-triazolo[1,5-c]chinazolin-5-on-2)-propionsäuremethylester, Fp. 253 -258
5. 3-(8-Trifluormethyl-1,2,4-triazolo[1,5-c]chinazolin-5-on-2)-propionsäureethylester, Fp. 220 - 222°C

### Beispiel 6

### 4-(1,2,4-Triazolo[1,5-c]chinazolin-5-on-2)-buttersäure

3,5 g Substanz des Beispiels 2 wurden in 70 ml 1 N Natronlauge bei Raumtemperatur über Nacht gerührt. Die Lösung wurde mit CH₂Cl₂ extrahiert, die Wasserphase mit 1 N Salzsäure auf pH 1 gestellt und der Niederschlag abgesaugt, mit Wasser gewaschen und getrocknet. Ausbeute: 2,8 g (84 %); Fp. 266 - 270°C.

In analoger Weise wurden Verbindungen hergestellt:

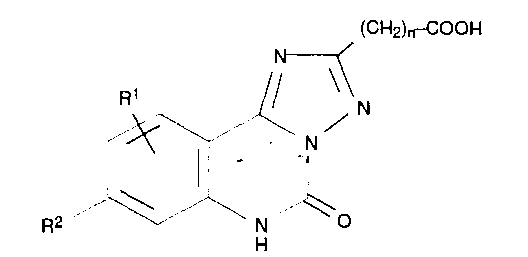

| Bsp. | R¹ | R² | n | Fp [°C] |
|---|---|---|---|---|
| 7 | H | H | 2 | 315 - 317 |
| 8 | 9-NO₂ | H | 2 | 250 - 254 |
| 9 | 9-NO₂ | H | 3 | 153 - 157 |
| 10 | H | H | 1 | 330 - 335 |
| 11 | 9-NO₂ | H | 1 | 315 - 320 |
| 12 | 9-Cl | H | 2 | 300 - 301 |
| 13 | H | NO₂ | 3 | 234 - 236 |
| 14 | H | NO₂ | 2 | 272 - 273 |
| 15 | 9-NO₂ | NO₂ | 2 | 224 - 226 |
| 16 | 9-CH₃ | CH₃ | 2 | > 350 |
| 17 | 9-NO₂ | CF₃ | 2 | 241 - 243 |
| 18 | H | CF₃ | 2 | 293 - 295 |
| 19 | 7,8-Benzo | | 2 | 313 - 319 |
| 20 | 8,9-Benzo | | 2 | 337 - 339 |

### Beispiel 21

### 3-(8,9-Dimethyl-1,2,4-triazolo[1,5-c]chinazolin-5-on-2)-propionsäurebenzylamid

1,7 g Säure des Beispiels 16 wurden mit 1,2 g 1-Hydroxybenztriazolhydrat und 1,4 g Dicyclohexylcarbodiimid in 15 ml DMF 90 min gerührt, anschließend eine Lösung von 0,65 g Benzylamin in 5 ml DMF zugegeben und weitere 15 h bei Raumtemperatur gerührt. Die Reaktionsmischung wurde filtriert, das Filtrat im Vakuum eingedampft und der Rückstand mit Methylenchlorid gewaschen. Ausbeute: 0,65 g (33%); Fp. 289 - 294°C.

### Beispiel 22

### 2-(9-Nitro-1,2,4-triazolo[1,5-c]chinazolin-5-on-2)-essigsäureethylester

1 g Ester (Beispiel 3) wurde 4 h in einer Mischung aus 10 g konzentrierter Schwefelsäure und 0,21 ml konzentrierter Salpetersäure gerührt. Die Mischung wurde auf Eis gegossen, der Niederschlag abgesaugt, mit Wasser gewaschen und getrocknet. Ausbeute: 0,75 g; Fp. 188 - 190°C.

In ähnlicher Weise wurden hergestellt:
23. 3-(9-Nitro-1,2,4-triazolo[1,5-c]chinazolin-5-on-2)-propionsäureethylester, Fp. 120 -126°C.
24. 4-(9-Nitro-1,2,4-triazolo[1,5-c]chinazolin-5-on-2)-buttersäuremethylester, Fp. 192 - 196°C.

### Beispiel 25

### 3-(8-Trifluormethyl-9-nitro-1,2,4-triazolo[1,5-c]chinazolin-5-on-2)-propionsaureethylester

5 g Ester (Beispiel 5) wurden portionsweise bei 0°C zu einer Mischung von 10 ml rauchender Salpetersäure und 14,5 ml konzentrierter Schwefelsäure gegeben. Nach 2 h wurde der Reaktionsansatz auf Eis gegossen, mit Essigester extrahiert, die organische Phase getrocknet, eingeengt und der Rückstand an Kieselgel mit Methylenchlorid / Methanol (50 : 1) chromatographiert. Ausbeute: 2,0 g; Fp. 166 - 168°C.

In analoger Weise wurde hergestellt:

### Beispiel 26

3-(8,9-Dinitro-1,2,4-triazolo[1,5-c]chinazolin-5-on-2)-propionsäureethylester, Fp. 170 - 172°C.

## Patentansprüche

1. Triazolochinazoline der Formel I 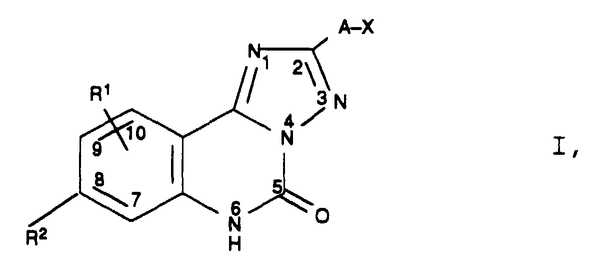 worin
A eine C₁₋₅-Alkylengruppe ist,
X eine Carboxylgruppe, die in Form ihres Salzes mit einem physiologisch verträglichen Amin- oder Metallkation vorliegen kann; den Rest 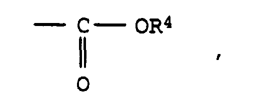 wobei R⁴ einen C₁₋₈-Alkylrest, eine Cycloalkylgruppe mit 3 bis 8 Kohlenstoffatomen im Ring, einen Benzylrest, einen der Reste -(CH₂)ₙ-O-R⁵ oder 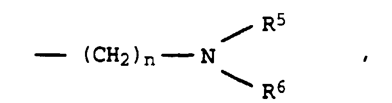 in denen n für die Zahl 2, 3 oder 4 und
R⁵ und R⁶ für eine C₁₋₃-Alkylgruppe stehen; eine Hydroxyl-, C₁₋₄-Hydroxyalkyl-, C₁₋₄-Alkylcarbonyl-, Nitrilo-C₁₋₄-alkyl-, Tetrazolyl- oder eine Carbonylaminotetrazolgruppe darstellt, und
R¹ und R², die gleich oder verschieden sein können, Wasserstoff-, Fluor-, Chlor- oder Bromatome, Trifluormethyl-, Cyano-, Nitro-, Amino-, C₁₋₅-Alkyl-, Mono- oder Di-C₁₋₅-alkylaminogruppen, einen C₁₋₆-Alkylthio-, C₁₋₆-Alkylsulfenyl-, C₁₋₆-Alkylsulfonyl-, Aminosulfonyl-, Di-C₁₋₆-alkylaminosulfonylrest bedeuten oder
R¹ und R² zusammen eine Methylen- oder Ethylendioxygruppe oder eine geradkettige C₃₋₅-Alkylengruppe bedeuten.

2. Verfahren zur Herstellung der Triazolochinazoline der Formel I gemäß Anspruch 1, dadurch gekennzeichnet, daß man
a) ein Hydrazinochinazolin der Formel II 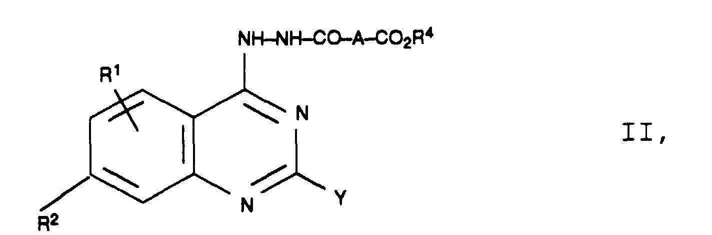 in der A, R¹ und R² die für Formel I angegebenen Bedeutungen haben, R⁴ ein C₁₋₈-Alkylrest, eine Cycloalkylgruppe mit 3 bis 8 Kohlenstoffatomen im Ring, ein Benzylring oder der Rest -(CH₂)-OR⁵ ist und Y eine Hydroxylgruppe oder ein Brom- oder Chloratom darstellt, intramolekular kondensiert und die so erhaltenen Ester gegebenenfalls anschließend umestert oder verseift und die freien Säuren gegebenenfalls mit einem Amin oder einem Metallkation in physiologisch verträgliche Salze überführt oder die freien Säuren zu den Hydroxyalkylverbindungen reduziert oder in die Nitrile, Tetrazolamino- und Carbamoylverbindungen überführt oder
b) in Verbindungen der Formel VII 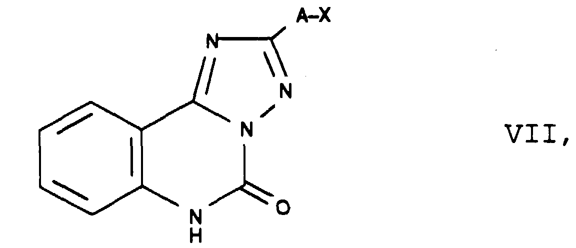 die Substituenten R¹ und/oder R² einführt.

3. Triazolochinazoline der Formel I gemäß Anspruch 1 zur Verwendung bei der Bekämpfung von Krankheiten.

4. 3-(7,8-Benzo-1,2,4-triazolo[1,5-c]chinazolin-5-on-2)-propionsäure

5. 3-(8,9-Benzo-1,2,4-triazolo[1,5-c]chinazolin-5-on-2)-propionsäure

## Claims

1. A triazoloquinazoline of the formula I 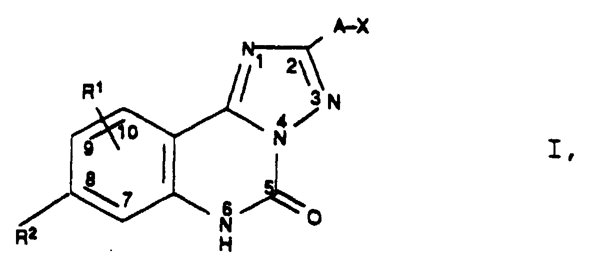 where
A is C₁-C₅-alkylene,
X is carboxyl which can be in the form of its salt with a physiologically tolerated amine or metal cation; the radical 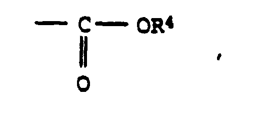 where R⁴ is C₁₋₈-alkyl, cycloalkyl with 3 to 8 carbon atoms in the ring, benzyl, one of the radicals -(CH₂)ₙ-O-R⁵ or 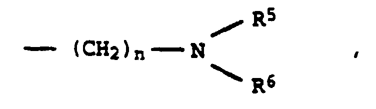 where n is the number 2, 3 or 4 and R⁵ and R⁶ are each C₁₋₃-alkyl, hydroxyl, C₁₋₄-hydroxyalkyl, C₁₋₄-alkylcarbonyl, nitrilo-C₁₋₄-alkyl, tetrazolyl or carbonylaminotetrazole, and
R¹ and R², which can be identical or different, are each hydrogen, fluorine, chlorine or bromine, trifluoromethyl, cyano, nitro, amino, C₁₋₅-alkyl, mono- or di-C₁₋₅-alkylamino, C₁₋₆-alkylthio, C₁₋₆-alkylsulfenyl, C₁₋₆-alkylsulfonyl, aminosulfonyl, di-C₁₋₆-alkylaminosulfonyl, or
R¹ and R² together are methylene- or ethylenedioxy or straight-chain C₃₋₅-alkylene.

2. A process for preparing a triazoloquinazoline of the formula I as claimed in claim 1, which comprises
a) intramolecular condensation of a hydrazinoquinazoline of the formula II 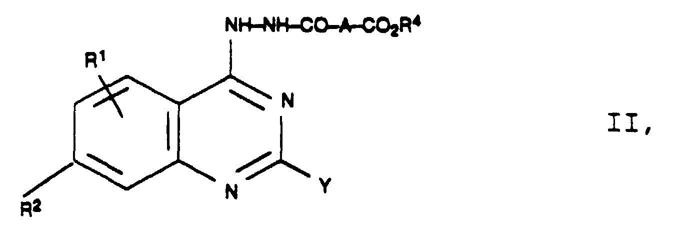 where A, R¹ and R² have the meanings stated for formula I, R⁴ is C₁₋₈-alkyl, cycloalkyl with 3 to 8 carbon atoms in the ring, a benzyl ring or the radical -(CH₂)-OR⁵, and Y is hydroxyl or bromine or chlorine, and subsequently transesterifying or hydrolyzing where appropriate the esters obtained in this way, and converting the free acid where appropriate with an amine or a metal cation into a physiologically tolerated salt, or reducing the free acid to the hydroxyalkyl compound or converting it into the nitrile, tetrazolamino or carbamoyl compound, or
b) introducing the substituents R¹ and/or R² into compounds of the formula VII

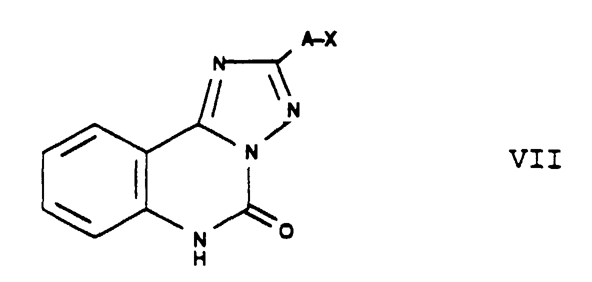

3. A triazoloquinazoline of the formula I as claimed in claim 1 for use for controlling diseases.

4. 3-(7,8-Benzo-1,2,4-triazolo[1,5-c]quinazolin-5-on-2yl)-propionic acid.

5. 3-(8,9-Benzo-1,2,4-triazolo[1,5-c]quinazolin-5-on-2yl)-propionic acid.

## Revendications

1. Triazoloquinazolines de formule I 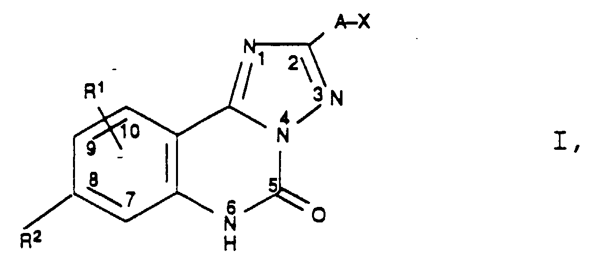 dans laquelle
A représente un groupe alkylène en C1-C5,
X représente un groupe carboxyle qui peut être à l'état de sel d'une amine ou d'un cation métallique acceptable pour les usages pharmaceutiques ; le groupe 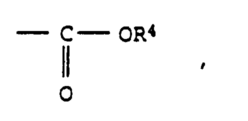 dans lequel R⁴ représente un groupe alkyle en C1-C8, un groupe cycloalkyle contenant 3 à 8 atomes de carbone cycliques, un groupe benzyle, l'un des groupes -(CH₂)ₙ-O-R⁵ ou 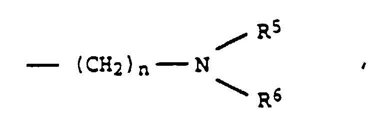 dans lesquels n est égal à 2, 3 ou 4 et
R⁵ et R⁶ représentent chacun un groupe alkyle en C1-C3, un groupe hydroxy, hydroxyalkyle en C1-C4, (alkyle en C1-C4)carbonyle, nitriloalkyle en C1-C4, tétrazolyle ou carbonylaminotétrazolyle, et
R¹ et R², qui peuvent avoir des significations identiques ou différentes, représentent chacun un atome d'hydrogène, de fluor, de chlore ou de brome, un groupe trifluorométhyle, cyano, nitro, amino, alkyle en C1-C5, mono- ou di-(alkyle en C1-C5)amino, alkylthio en C1-C6, alkylsulfényle en C1-C6, alkylsulfonyle en C1-C6, aminosulfonyle, di-(alkyle en C1-C6)aminosulfonyle, ou bien
R¹ et R² forment ensemble un groupe méthylène- ou éthylène-dioxy ou un groupe alkylène à chaîne droite en C3-C5.

2. Procédé de préparation des triazoloquinazolines de formule I de la revendication 1, caractérisé par le fait que
a) on soumet une hydrazinoquinazoline de formule II 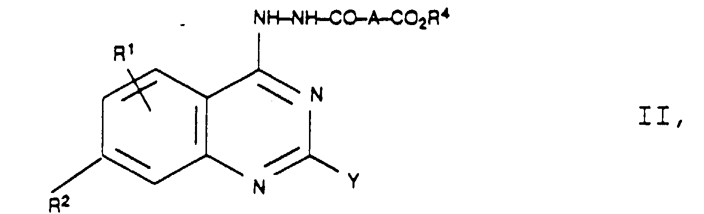 dans laquelle A, R¹ et R² ont les significations indiquées en référence à la formule I, R⁴ représente un groupe alkyle en C1-C8, un groupe cycloalkyle contenant 3 à 8 atomes de carbone cycliques, un groupe benzyle ou le groupe -(CH₂)-OR⁵ et Y représente un groupe hydroxy ou un atome de brome ou de chlore, à une condensation intramoléculaire, ce qui donne des esters que, le cas échéant, on transestérifie ou on saponifie, ce qui donne les acides libres qu'on convertit le cas échéant, à l'aide d'une amine ou d'un cation métallique, en sels acceptables pour l'usage pharmaceutique, ou bien qu'on réduit en les dérivés hydroxyalkyliques ou bien qu'on convertit en les nitriles, les dérivés tétrazolaminés et carbamoylés, ou bien
b) dans des composés de formule VII 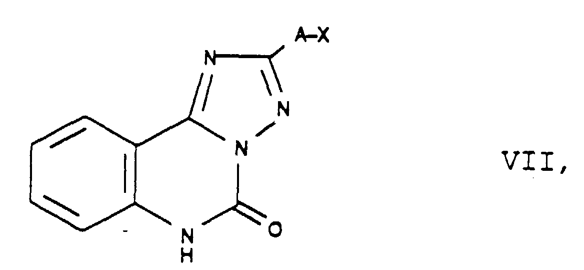 on introduit les substituants R¹ et/ou R².

3. Triazoloquinazolines de formule I de la revendication 1, pour l'utilisation dans le traitement de maladies.

4. L'acide 3-(7,8-benzo-1,2,4-triazolo[1,5-c]quinazoline-5-one-2)-propionique.

5. L'acide 3-(8,9-benzo-1,2,4-triazolo[1,5-c]quinazoline-5-one-2)-propionique.
